# EUROPÄISCHE PATENTSCHRIFT

(11) **EP 2 504 378 B1**
(45) Veröffentlichungstag und Bekanntmachung des Hinweises auf die Patenterteilung: **29.07.2015**
(21) Anmeldenummer: 10778672.5
(22) Anmeldetag: 22.11.2010
(51) Int. Cl.: C08G 63/46, C08G 63/91, A61K 8/85

(54) **VERWENDUNG VON HOCHVERZWEIGTEN POLYESTERN IN KOSMETISCHEN UND DERMATOLOGISCHEN FORMULIERUNGEN**
USE OF HYPERBRANCHED POLYESTERS IN COSMETIC AND DERMATOLOGICAL FORMULATIONS
UTILISATION DE POLYESTERS TRÈS RAMIFIÉS DANS DES FORMULATIONS COSMÉTIQUES ET DERMATOLOGIQUES

(30) Priorität: 26.11.2009 EP 09177210
(43) Veröffentlichungstag der Anmeldung: 03.10.2012
(73) Patentinhaber: BASF SE, 67056 Ludwigshafen (DE)
(72) Erfinder: WENDEL, Volker, 64342 Seeheim-Jugenheim (DE); LAUBENDER, Matthias, 67105 Schifferstadt (DE); STUMBE, Jean-François, F-67200 Strasbourg (FR); BRUCHMANN, Bernd, 67251 Freinsheim (DE)
(86) Internationale Anmeldenummer: PCT/EP2010/067872
(87) Internationale Veröffentlichungsnummer: WO 2011/064153

(56) Entgegenhaltungen:
- EP-A2- 1 557 441
- US-A1- 2008 038 215
- US-A1- 2008 153 931
- US-A1- 2008 274 149

## Beschreibung

Die vorliegende Erfindung betrifft die Verwendung von substituierten hochverzweigten Polyestern als Verdicker in der Kosmetik und der Dermatologie.

Verdickungsmittel werden auf dem Gebiet der Pharmazie und der Kosmetik in großem Umfang zur Erhöhung der Viskosität von Zubereitungen eingesetzt.

Das Verdickungsmittel wird danach ausgewählt, ob die Zubereitung wässrig, ölig oder tensidisch ist. Eine Übersicht hierzu gibt Hugo Janistyn, Handbuch der Kosmetika und Riechstoffe, Hüthig Verlag Heidelberg, Band 1, 3. Auflage, 1978, S. 979.

Beispiele für häufig verwendete Verdickungsmittel für wässrige Lösungen sind Fettsäurepolyethylenglycolmonoester, Fettsäurepolyethylenglycoldiester, Fettsäurealkanolamide, oxethylierte Fettalkohole, ethoxylierte Glycerinfettsäureester, Celluloseether, Natriumalginat, Polyacrylsäuren sowie Neutralsalze.

Auch Carboxylgruppen enthaltende Polymere sind als Verdicker bekannt. Dazu zählen Homo- und Copolymere von monoethylenisch ungesättigten Carbonsäuren wie Acrylsäure, Methacrylsäure, Maleinsäure, Maleinsäureanhydrid und Itaconsäure. Diese Polymere sind häufig wenigstens zu einem geringen Anteil vernetzt. Solche Polymere sind beispielsweise in US 2,798,053, US 3,915,921, US 3,940,351 , US 4,062,817, US 4,066,583, US 4,267,103, US 5,349,030 und US 5,373,044 beschrieben.

Häufige Nachteile dieser Polymere bei der Verwendung als Verdicker sind deren pH-Abhängigkeit und hydrolytische Instabilität. Weiterhin werden häufig große Mengen der Polymere benötigt, um den erwünschten Verdickungs-Effekt zu erzielen und die Stabilität der Zubereitungen bei Anwesenheit von Elektrolyten ist gering.

Auch natürlich vorkommende Materialien wie Casein, Alginate, Methylcellulose, Hydroxyethylcellulose, Hydroxypropylcellulose und Carbomethoxycellulose werden als Verdicker eingesetzt. Diese haben u.a. den Nachteil der Empfindlichkeit gegenüber mikrobiologischen Faktoren und es bedarf folglich des Zusatzes von Bioziden.

Typische Verdicker von öligen Zubereitungen, im folgenden auch Ölverdicker genannt, sind Metallseifen, amorphes Siliciumdioxid, Hydroxystearin, Verbindungen quaternärer Ammoniumbasen mit Bentoniten, Wachse und Paraffine.

Tensidlösungen werden beispielsweise verdickt durch Fettsäurealkylolamide, Aminoxide, Cellulosederivate, Polysaccharide und die oben genannten Carboxylgruppen enthaltende Polymere.

Hochfunktionelle hochverzweigte Polyester und Verfahren zu deren Herstellung sind beispielsweise in DE 101 63 163, DE 102 19 508, DE 102 40 817, DE 103 48 463, DE 10 2004 026904 und DE 10 2005 060783 beschrieben.

WO 2006/018063 beschreibt Zusammensetzungen für die Haarkosmetik, die hydrophob funktionalisierte dendritische Makromoleküle enthalten. Die dendritischen Makromoleküle sind entweder aus Polyestereinheiten (erhältlich unter dem Handelsnamen Boltorn) oder aus Polyamideinheiten (erhältlich unter dem Handelsnamen Hybrane) aufgebaut.

DE 10 2005 063 096 beschreibt kosmetische Mittel, die 0,05 bis 20 Gew.-% mindestens eines hyperverzweigten Polyesters und/oder Polyesteramides enthalten. Die Mittel sollen haarreinigende und/oder haarpflegende Eigenschaften aufweisen. Die Polyester und/oder Polyesteramide sind nicht substituiert.

In der WO 2004/078809 werden hochverzweigte Polymere und diese enthaltende kosmetische Zusammensetzungen offenbart.

Eine Aufgabe der vorliegenden Erfindung war es, rheologiemodifizierende, insbesondere verdickende, insbesondere ölverdickende Polymere zu finden, die für kosmetische Anwendungen gut geeignet sind und insbesondere im Gebiet Hautkosmetik gute anwendungstechnische Eigenschaften besitzen. Dazu gehören neben der guten Verdickerwirkung bei geringem Materialeinsatz auch Klarheit bei Gelanwendungen, (co-) emulgierende und stabilisierende Wirkung für ölunlösliche und/oder schwer zu stabilisierende Komponenten, gute Einarbeitbarkeit in kosmetische Zubereitungen. Insbesondere für Gele ist eine möglichst hohe Transparenz (Klarheit) der Zubereitungen erwünscht. Um eine breitestmögliche Formulierbarkeit zu gewährleisten ist es erwünscht, dass die Verdicker farb- und geruchsarm, idealerweise farb- und geruchslos sind.

Zudem ist es bei der Verwendung in (haut)kosmetischen und/oder dermatologischen Anwendungen notwendig, dass keine allergenen Reaktionen ausgelöst werden.

Die Aufgabe wird durch die nachstehend beschriebenen substituierten hochverzweigten Polyester gelöst.

Unter hochverzweigten Polyestern werden im Rahmen dieser Erfindung unvernetzte Makromoleküle mit Hydroxyl- und Carboxylgruppen verstanden, die sowohl strukturell als auch molekular uneinheitlich sind. Sie können auf der einen Seite ausgehend von einem Zentralmolekül analog zu Dendrimeren, jedoch mit uneinheitlicher Kettenlänge der Äste aufgebaut sein. Sie können auf der anderen Seite auch linear, mit funktionellen Seitengruppen, aufgebaut sein oder aber, als Kombination der beiden Extreme, lineare und verzweigte Molekülteile aufweisen. Zur Definition von dendrimeren und hyperverzweigten Polymeren siehe auch P.J. Flory, J. Am. Chem. Soc. 1952, 74, 2718 und H. Frey et al., Chem. Eur. J. 2000, 6, No. 14, 2499.

Unter "hochverzweigt" wird im Zusammenhang mit der vorliegenden Erfindung verstanden, dass der Verzweigungsgrad (Degree of Branching, DB), dass heißt die mittlere Anzahl dendritischer Verknüpfungen plus mittlere Anzahl der Endgruppen pro Molekül geteilt durch die Summe der mittleren Anzahl der dendritischen Verknüpfungen, der mittleren Anzahl der linearen Verknüpfungen und der mittleren Anzahl der Endgruppen, multipliziert mit 100 , 10 bis 99.9 %, bevorzugt 20 bis 99 %, besonders bevorzugt 20 - 95 % beträgt.

Aus der Literatur ist neben dem Ausdruck hochverzweigt auch der Ausdruck hyperverzweigt bekannt. Im Rahmen der vorliegenden Erfindung sollen die beiden Ausdrücke synonym verstanden werden.

Unter "dendrimer" wird im Zusammenhang mit der vorliegenden Erfindung verstanden, daß der Verzweigungsgrad 99,9 - 100% beträgt. Zur Definition des "Degree of Branching" siehe H. Frey et al., Acta Polym. 1997, 48, 30.

Unvernetzt im Rahmen dieser Schrift bedeutet, daß ein Vernetzungsgrad von weniger als 15 Gew.%, bevorzugt von weniger als 10 Gew.%, bestimmt über den unlöslichen Anteil des Polymeren, vorhanden ist.
Der unlösliche Anteil des Polymeren wurde bestimmt durch vierstündige Extraktion mit dem gleichen Lösungsmittel, wie es für die Gelpermeationschromatographie verwendet wird, also Tetrahydrofuran, Dimethylacetamid oder Hexafluorisopropanol, je nachdem, in welchem Lösungsmittel das Polymer besser löslich ist, in einer Soxhlet-Apparatur und nach Trocknung des Rückstandes bis zur Gewichtskonstanz Wägung des verbliebenen Rückstandes.

Die hochverzweigten Polyester werden wie nachfolgend beschrieben hergestellt.

Mindestens eine aliphatische, cycloaliphatische, araliphatische oder aromatische Dicarbonsäure (A₂) oder Derivate derselben und gegebenenfalls ein zweiwertiger aliphatischer, cycloaliphatischer, araliphatischer oder aromatischer Alkohol (B₂), welcher 2 OH-Gruppen aufweist, werden mit
mindestens einem x-wertigen aliphatischen, cycloaliphatischen, araliphatischen oder aromatischen Alkohol (Cₓ), der mehr als zwei OH-Gruppen aufweist und x eine Zahl größer 2, vorzugsweise zwischen 3 und 8, besonders bevorzugt zwischen 3 und 6, ganz besonders bevorzugt von 3 bis 4 und insbesondere 3 darstellt,
gegebenenfalls in Gegenwart weiterer funktionalisierter Bausteine E umgesetzt,
wobei man das Verhältnis der reaktiven Gruppen im Reaktionsgemisch so wählt, dass man ein molares Verhältnis von OH-Gruppen zu Carboxylgruppen oder deren Derivaten von 5:1 bis 1:5, vorzugsweise von 4:1 bis 1:4, besonders bevorzugt von 3:1 bis 1:3 und ganz besonders bevorzugt von 2:1 bis 1:2 einstellt.

Zu den Dicarbonsäuren (A₂) gehören beispielsweise aliphatische Dicarbonsäuren, wie Oxalsäure, Malonsäure, Bernsteinsäure, Glutarsäure, Adipinsäure, Pimelinsäure, Korksäure, Azelainsäure, Sebacinsäure, Undecan-α,ω-dicarbonsäure, Dodecan-α,ω-dicarbonsäure, cis- und trans-Cyclohexan-1,2-dicarbonsäure, cis- und trans-Cyclohexan-1,3-dicarbonsäure, cis- und trans-Cyclohexan-1,4-dicarbonsäure, cis- und trans-Cyclopentan-1,2-dicarbonsäure, cis- und trans-Cyclopentan-1,3-dicarbonsäure. Weiterhin können auch aromatische Dicarbonsäuren, wie zum Beispiel Phthalsäure, Isophthalsäure oder Terephthalsäure verwendet werden. Auch ungesättigte Dicarbonsäuren, wie Maleinsäure oder Fumarsäure sind einsetzbar.

Die genannten Dicarbonsäuren können auch substituiert sein mit einem oder mehreren Resten, ausgewählt aus

C₁-C₁₀-Alkylgruppen, beispielsweise Methyl, Ethyl, n-Propyl, iso-Propyl, n-Butyl, isoButyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, 2-Ethylhexyl, Trimethylpentyl, n-Nonyl oder n-Decyl,
C₃-C₁₂-Cycloalkylgruppen, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
Alkylengruppen wie Methylen oder Ethyliden oder

C₆-C₁₄-Arylgruppen wie beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl.

Als beispielhafte Vertreter für substituierte Dicarbonsäuren seien genannt: 2-Methylmalonsäure, 2-Ethylmalonsäure, 2-Phenylmalonsäure, 2-Methylbernsteinsäure, 2-Ethylbernsteinsäure, 2-Phenylbernsteinsäure, Itaconsäure, 3,3-Dimethylglutarsäure.

Weiterhin lassen sich Gemische von zwei oder mehreren der vorgenannten Dicarbonsäuren einsetzen.

Die Dicarbonsäuren lassen sich entweder als solche oder in Form von Derivaten einsetzen.

Unter Derivaten werden bevorzugt verstanden
- die betreffenden Anhydride in monomerer oder auch polymerer Form,
- Mono- oder Dialkylester, bevorzugt Mono- oder Di-C₁-C₄-alkylester, besonders bevorzugt Mono- oder Dimethylester oder die entsprechenden Mono- oder Diethylester,
- ferner Mono- und Divinylester sowie
- gemischte Ester, bevorzugt gemischte Ester mit unterschiedlichen C₁-C₄-Alkylkomponenten, besonders bevorzugt gemischte Methylethylester.

C₁-C₄-Alkyl bedeutet im Rahmen dieser Schrift Methyl, Ethyl, *iso*-Propyl, n-Propyl, n-Butyl, *iso*-Butyl, *sek*-Butyl und *tert*-Butyl, bevorzugt Methyl, Ethyl und n-Butyl, besonders bevorzugt Methyl und Ethyl und ganz besonders bevorzugt Methyl.

Im Rahmen der vorliegenden Erfindung ist es auch möglich, ein Gemisch aus einer Dicarbonsäure und einem oder mehreren ihrer Derivate einzusetzen. Gleichfalls ist es im Rahmen der vorliegenden Erfindung möglich, ein Gemisch mehrerer verschiedener Derivate von einer oder mehreren Dicarbonsäuren einzusetzen.

Besonders bevorzugt setzt man Malonsäure, Bernsteinsäure, Glutarsäure, Adipinsäure, 1,2-, 1,3- oder 1,4-Cyclohexandicarbonsäure (Hexahydrophthalsäuren), Phthalsäure, Isophthalsäure, Terephthalsäure oder deren Mono- oder Dialkylester ein.

Als Diole (B₂) gemäß der vorliegenden Erfindung verwendet man beispielsweise Ethylenglykol, Propan-1,2-diol, Propan-1,3-diol, Butan-1,2-diol, Butan-1,3-diol, Butan-1,4-diol, Butan-2,3-diol, Pentan-1,2-diol, Pentan-1,3-diol, Pentan-1,4-diol, Pentan-1,5-diol, Pentan-2,3-diol, Pentan-2,4-diol, Hexan-1,2-diol, Hexan-1,3-diol, Hexan-1,4-diol, Hexan-1,5-diol, Hexan-1,6-diol, Hexan-2,5-diol, Heptan-1,2-diol 1,7-Heptandiol, 1,8-Octandiol, 1,2-Octandiol, 1,9-Nonandiol, 1,2-Decandiol, 1,10-Decandiol, 1,2-Dodecandiol, 1,12-Dodecandiol, 1,5-Hexadien-3,4-diol, 1,2- und 1,3-Cyclopentandiole, 1,2-, 1,3- und 1,4-Cyclohexandiole, 1,1-, 1,2-, 1,3- und 1,4-Bis-(Hydroxymethyl)cyclohexane, 1,1-, 1,2-, 1,3- und 1,4-Bis(Hydroxyethyl)cyclohexane, Neopentylglykol, (2)-Methyl-2,4-pentandiol, 2,4-Dimethyl-2,4-Pentandiol, 2-Ethyl-1,3-hexandiol, 2,5-Dimethyl-2,5-hexandiol, 2,2,4-Trimethyl-1,3-pentandiol, Pinacol, Diethylenglykol, Triethylenglykol, Dipropylenglykol, Tripropylenglykol, Polyethylenglykole HO(CH₂CH₂O)ₙ-H oder Polypropylenglykole HO(CH[CH₃]CH₂O)ₙ-H, wobei n eine ganze Zahl und n ≥ 4 ist, Polyethylen-polypropylenglykole, wobei die Abfolge der Ethylenoxid- der Propylenoxid-Einheiten blockweise oder statistisch sein kann, Polytetramethylenglykole, vorzugsweise bis zu einem Molgewicht bis zu 5000 g/mol, Poly-1,3-Propandiole, vorzugsweise mit einem Molgewicht bis zu 5000 g/mol, Polycaprolactone oder Gemische von zwei oder mehr Vertretern der voranstehenden Verbindungen. Dabei kann eine oder auch beide Hydroxylgruppen in den vorstehend genannten Diolen durch SH-Gruppen substituiert werden. Bevorzugt eingesetzte Diols sind Ethylenglykol, 1,2-Propandiol, 1,3-Propandiol, 1,4-Butandiol, 1,5-Pentandiol, 1,6-Hexandiol, 1,8-Octandiol, 1,2-, 1,3- und 1,4-Cyclohexandiol, 1,3- und 1,4-Bis(hydroxymethyl)cyclohexan, sowie Diethylenglykol, Triethylenglykol, Dipropylenglykol und Tripropylenglykol.

Die zweiwertigen Alkohole B₂ können optional noch weitere Funktionalitäten wie beispielsweise Carbonyl, Carboxy, Alkoxycarbonyl oder Sulfonyl enthalten, wie beispielsweise Dimethylolpropionsäure oder Dimethylolbuttersäure, sowie deren C₁-C₄-Alkylester, bevorzugt weisen die Alkohole B₂ jedoch keine weiteren Funktionalitäten auf.

Mindestens trifunktionelle Alkohole (Cₓ) umfassen Glycerin, Trimethylolmethan, Trimethylolethan, Trimethylolpropan, 1,2,4-Butantriol, Tris(hydroxymethyl)amin, Tris(hydroxyethyl)amin, Tris(hydroxypropyl)amin, Pentaerythrit, Diglycerin, Triglycerin oder höhere Kondensationsprodukte des Glycerins, Di(trimethylolpropan), Di(pentaerythrit), Trishydroxymethylisocyanurat, Tris(hydroxyethyl)isocyanurat (THEIC), Tris(hydroxypropyl)isocyanurat, Inositole oder Zucker, wie zum Beispiel Glucose, Fructose oder Sucrose, Zuckeralkohole wie z.B. Sorbit, Mannit, Threit, Erythrit, Adonit (Ribit), Arabit (Lyxit), Xylit, Dulcit (Galactit), Maltit, Isomalt, tri- oder höherfunktionelle Polyetherole auf Basis tri- oder höherfunktioneller Alkohole und Ethylenoxid, Propylenoxid und/oder Butylenoxid.

Dabei sind Glycerin, Diglycerin, Triglycerin, Trimethylolethan, Trimethylolpropan, 1,2,4-Butantriol, Pentaerythrit, Tris(hydroxyethyl)isocyanurat sowie deren Polyetherole auf Basis von Ethylenoxid und/oder Propylenoxid besonders bevorzugt.

Das erfindungsgemäße Verfahren kann in Substanz oder in Gegenwart eines Lösemittels durchgeführt werden. Als Lösemittel geeignet sind beispielsweise Kohlenwasserstoffe wie Paraffine oder Aromaten. Besonders geeignete Paraffine sind n-Heptan und Cyclohexan. Besonders geeignete Aromaten sind Toluol, ortho-Xylol, meta-Xylol, para-Xylol, Xylol als Isomerengemisch, Ethylbenzol, Chlorbenzol und ortho- und meta-Dichlorbenzol. Weiterhin sind als Lösemittel in Abwesenheit von sauren Katalysatoren ganz besonders Ether geeignet, wie beispielsweise Dioxan oder Tetrahydrofuran und Ketone wie beispielsweise Methylethylketon und Methylisobutylketon.

Die Menge an zugesetztem Lösemittel beträgt erfindungsgemäß mindestens 0,1 Gew.-%, bezogen auf die Masse der eingesetzten umzusetzenden Ausgangsmaterialien, bevorzugt mindestens 1 Gew.-% und besonders bevorzugt mindestens 10 Gew.-%. Man kann auch Überschüsse an Lösemittel, bezogen auf die Masse an eingesetzten umzusetzenden Ausgangsmaterialien, einsetzen, beispielsweise das 1,01 bis 10-fache. Lösemittel-Mengen von mehr als dem 100-fachen, bezogen auf die Masse an eingesetzten umzusetzenden Ausgangsmaterialien, sind nicht vorteilhaft, weil bei deutlich niedrigeren Konzentrationen der Reaktionspartner die Reaktionsgeschwindigkeit deutlich nachlässt, was zu unwirtschaftlichen langen Umsetzungsdauern führt.

In einer bevorzugten Ausführungsform wird die Reaktion frei von Lösungsmittel durchgeführt.

Zur Durchführung des erfindungsgemäßen Verfahrens kann man in Gegenwart eines Wasser entziehenden Mittels als Additiv arbeiten, das man zu Beginn der Reaktion zusetzt. Geeignet sind beispielsweise Molekularsiebe, insbesondere Molekularsieb 4Å, MgSO₄ und Na₂SO₄. Man kann auch während der Reaktion weiteres Wasser entziehendes Mittel zufügen oder Wasser entziehendes Mittel durch frisches Wasser entziehendes Mittel ersetzen. Man kann auch während der Reaktion gebildetes Wasser bzw. Alkohol abdestillieren und beispielsweise einen Wasserabscheider einsetzen, bei dem das Wasser mit Hilfe eines Schleppmittels entfernt wird.

Weiterhin kann die Abtrennung durch Strippen erfolgen, beispielsweise durch Durchleiten eines unter den Reaktionsbedingungen inerten Gases durch das Reaktionsgemisch erfolgen, gegebenenfalls zusätzlich zu einer Destillation. Als Inertgase eignen sich vorzugsweise Stickstoff, Edelgase, Kohlendioxid oder Verbrennungsgase.

Man kann das erfindungsgemäße Verfahren in Abwesenheit von Katalysatoren durchführen. Vorzugsweise arbeitet man jedoch in Gegenwart von mindestens einem Katalysator. Die sind bevorzugt saure anorganische, metallorganische oder organische Katalysatoren oder Gemische aus mehreren sauren anorganischen, metallorganischen oder organischen Katalysatoren.

Als saure anorganische Katalysatoren im Sinne der vorliegenden Erfindung sind beispielsweise Schwefelsäure, Sulfate und Hydrogensyulfate, wie Natriumhydrogensulfat, Phosphorsäure, Phosphonsäure, hypophosphorige Säure, Aluminiumsulfathydrat, Alaun, saures Kieselgel (pH ≤ 6, insbesondere ≤ 5) und saures Aluminiumoxid zu nennen. Weiterhin sind beispielsweise Alumiumverbindungen der allgemeinen Formel Al(OR¹)₃ und Titanate der allgemeinen Formel Ti(OR¹)₄ als saure anorganische Katalysatoren einsetzbar, wobei die Reste R¹ jeweils gleich oder verschieden sein können und unabhängig voneinander gewählt sind aus

C₁-C₂₀-Alkylresten, beispielsweise Methyl, Ethyl, n-Propyl, iso-Propyl, n-Butyl, isoButyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, 2-Ethylhexyl, n-Nonyl, n-Decyl, n-Dodecyl, n-Hexadecyl oder n-Octadecyl.

C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl.

Bevorzugt sind die Reste R¹ in Al(OR¹)₃ bzw. Ti(OR¹)₄ jeweils gleich und gewählt aus n-Butyl, Isopropyl oder 2-Ethylhexyl.

Bevorzugte saure metallorganische Katalysatoren sind beispielsweise gewählt aus Dialkylzinnoxiden R¹₂SnO oder Dialkylzinnestern R¹₂Sn(OR²)₂ wobei R¹ wie oben stehend definiert ist und gleich oder verschieden sein kann.

R² kann die gleichen Bedeutungen haben wie R¹ und zusätzlich C₆-C₁₂-Aryl sein, beispielsweise Phenyl, o-, m- oder p-Tolyl, Xylyl oder Naphthyl. R² kann jeweils gleich oder verschieden sein.

Beispiele sind für zinnorganische Katalysatoren sind Zinn(II)-n-octanoat, Zinn-(II)-2-ethylhexanoat, Zinn-(II)-laurat, Dibutylzinnoxid, Diphenylzinnoxid, Dibutylzinndichlorid, Dibutylzinndiacetat, Dibutylzinndilaurat, Dibutylzinndimaleat oder Dioctylzinndiacetat.

Besonders bevorzugte Vertreter für saure metallorganische Katalysatoren sind Dibutylzinnoxid, Diphenylzinnoxid und Dibutylzinndilaurat.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugt sind Sulfonsäuren wie beispielsweise para-Toluolsulfonsäure. Man kann auch saure Ionentauscher als saure organische Katalysatoren einsetzen, beispielsweise Sulfonsäuregruppen-haltige Polystyrolharze, die mit etwa 2 mol-% Divinylbenzol vernetzt sind.

Man kann auch Kombinationen von zwei oder mehreren der vorgenannten Katalysatoren einsetzen. Auch ist es möglich, solche organische oder metallorganische oder auch anorganische Katalysatoren, die in Form diskreter Moleküle vorliegen, in immobilisierter Form, zum Beispiel an Kieselgel oder an Zeolithen, einzusetzen.

Wünscht man saure anorganische, metallorganische oder organische Katalysatoren einzusetzen, so setzt man erfindungsgemäß 0,1 bis 10 Gew.-%, bevorzugt 0,2 bis 2 Gew.-% Katalysator ein.

Enzyme oder Zersetzungsprodukte von Enzymen gehören ebenfalls zu den organischen Katalysatoren im Sinne der vorliegenden Erfindung. Besonders bevorzugt sind effektiven Menge einer Lipase, die erhältlich ist zum Beispiel aus Candida cylindracea, Candida lipolytica, Candida rugosa, Candida antarctica, Candida utilis, Chromobacterium viscosum, Geotrichum viscosum, Geotrichum candidum, Mucorjavanicus, Mucor mihei, pig pancreas, pseudomonas spp., pseudomonas fluoprescens, Pseudomonas cepacia, Rhizopus arrhizus, Rhizopus delemar, Rhizopus niveus, Rhizopus oryzae, Aspergillus niger, Penicillium roquefortii, Penicillium camembertii oder Esterase von Bacillus spp. Bacillus thermoglucosidasius.

Das erfindungsgemäße Verfahren wird vorzugsweise unter Inertgasatmosphäre, d.h. einem unter den Reaktionsbedingungen inerten Gas, durchgeführt, beispielsweise unter Kohlendioxid, Verbrennungsgasen, Stickstoff oder Edelgas, unter denen insbesondere Argon zu nennen ist.

Das erfindungsgemäße Verfahren wird bei Temperaturen von 60 bis 250°C durchgeführt. Vorzugsweise arbeitet man bei Temperaturen von 80 bis 200, besonders bevorzugt bei 100 bis 180°C.

Die Druckbedingungen des erfindungsgemäßen Verfahrens sind in der Regel unkritisch. Man kann bei deutlich verringertem Druck arbeiten, beispielsweise bei 10 bis 500 mbar. Das erfindungsgemäße Verfahren kann auch bei Drucken oberhalb von 500 mbar durchgeführt werden. Bevorzugt ist aus Gründen der Einfachheit die Umsetzung bei Atmosphärendruck; möglich ist aber auch eine Durchführung bei leicht erhöhtem Druck, beispielsweise bis 1200 mbar. Man kann auch unter deutlich erhöhtem Druck arbeiten, beispielsweise bei Drucken bis 10 bar. Bevorzugt ist die Umsetzung bei verringertem oder Atmosphärendruck, besonders bevorzugt bei Atmosphärendruck.

Die Umsetzungsdauer des erfindungsgemäßen Verfahrens beträgt üblicherweise 10 Minuten bis 48 Stunden, bevorzugt 30 Minuten bis 24 Stunden und besonders bevorzugt 1 bis 12 Stunden.

Nach beendeter Reaktion lassen sich die hochfunktionellen hoch- und hyperverzweigten Polyester leicht isolieren, beispielsweise durch Abfiltrieren des Katalysators und gegebenenfalls Abziehen des Lösemittels, wobei man das Abziehen des Lösemittels üblicherweise bei vermindertem Druck durchführt. Weitere gut geeignete Aufarbeitungsmethoden sind Ausfällen des Polymeren nach Zugabe von Wasser und anschließendes Waschen und Trocknen.
d) Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew%, bevorzugt 0,5 bis 25 Gew%, besonders bevorzugt 1 - 10 Gew% bei Temperaturen von beispielsweise 10 bis 200°C, bevorzugt 20 bis 180 °C und besonders bevorzugt 30 bis 160 °C unterworfen werden.

Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

Die Entfärbung des Reaktionsgemisches kann an beliebiger Stelle des Aufarbeitungsverfahrens erfolgen, beispielsweise auf der Stufe des rohen Reaktionsgemisches oder nach gegebenenfalls erfolgter Vorwäsche, Neutralisation, Wäsche oder Lösungsmittelentfernung.

Das Reaktionsgemisch kann weiterhin einer Vorwäsche e) und/oder einer Neutralisation f) und/oder einer Nachwäsche g) unterworfen werden, bevorzugt lediglich einer Neutralisation f). Gegebenenfalls können Neutralisation f) und Vorwäsche e) in der Reihenfolge auch vertauscht werden.

Aus der wäßrigen Phase der Wäschen und/oder Neutralisation können enthaltene Wertprodukte durch Ansäuern und Extraktion mit einem Lösungsmittel zumindest teilweise wiedergewonnen und von Neuem eingesetzt werden.

Zur Vor- oder Nachwäsche wird das Reaktionsgemisch in einem Waschapparat mit einer Waschflüssigkeit, beispielsweise Wasser oder einer 5 - 30 Gew%-igen, bevorzugt 5 - 20, besonders bevorzugt 5 - 15 Gew%-igen Kochsalz-, Kaliumchlorid-, Ammoniumchlorid-, Natriumsulfat- oder Ammoniumsulfatlösung, bevorzugt Wasser oder Kochsalzlösung, behandelt.

Das Mengenverhältnis Reaktionsgemisch : Waschflüssigkeit beträgt in der Regel 1: 0,1 - 1, bevorzugt 1 : 0,2 - 0,8, besonders bevorzugt 1 : 0,3 - 0,7.

Die Wäsche oder Neutralisation kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche oder Neutralisation im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Vorzugsweise werden Siebboden- oder gepackte beziehungsweise Füllkörperkolonnen, Rührbehälter oder Mixer-Settler-Apparate, sowie gepulste Kolonnen oder solche mit rotierenden Einbauten eingesetzt.

Die Vorwäsche wird bevorzugt dann eingesetzt, wenn Metallsalze, bevorzugt zinnorganische Verbindungen als Katalysator (mit)verwendet werden.

Eine Nachwäsche kann zur Entfernung von Base- oder Salzspuren aus dem neutralisierten Reaktionsgemisch vorteilhaft sein.

Zur Neutralisation f) kann das gegebenenfalls vorgewaschene Reaktionsgemisch, das noch geringe Mengen an Katalysator und/oder Carbonsäure enthalten kann, mit einer 5 - 25, bevorzugt 5 - 20, besonders bevorzugt 5 - 15 Gew%igen wäßrigen Lösung einer Base, wie beispielsweise Alkali- oder Erdalkalimetalloxide, -hydroxide, -carbonate oder -hydrogencarbonate, bevorzugt Natronlauge, Kalilauge, Natriumhydrogencarbonat, Natriumcarbonat, Kaliumhydrogencarbonat, Kalziumhydroxid, Kalkmilch, Ammoniak, Ammoniakwasser oder Kaliumcarbonat, der gegebenenfalls 5 - 15 Gew% Kochsalz, Kaliumchlorid, Ammoniumchlorid oder Ammoniumsulfat zugesetzt sein können, besonders bevorzugt mit Natronlauge oder Natronlauge-Kochsalz-Lösung, neutralisiert werden. Der Neutralisationsgrad beträgt bevorzugt 5 bis 60 Mol%, vorzugsweise 10 bis 40 Mol%, besonders bevorzugt 20 bis 30 Mol%, bezogen auf die Säuregruppen enthaltenden Monomere.

Die Zugabe der Base erfolgt in einer Weise, daß die Temperatur im Apparat nicht über 60 °C ansteigt, bevorzugt zwischen 20 und 35 °C beträgt und der pH-Wert 4 - 13 beträgt. Die Abfuhr der Neutralisationswärme erfolgt vorzugsweise durch Kühlung des Behälters mit Hilfe von innenliegenden Kühlschlangen oder über eine Doppelwandkühlung.

Das Mengenverhältnis Reaktionsgemisch : Neutralisationsflüssigkeit beträgt in der Regel 1: 0,1 - 1, bevorzugt 1 : 0,2 - 0,8, besonders bevorzugt 1 : 0,3 - 0,7.

Hinsichtlich der Apparatur gilt das oben Gesagte.
h) Falls ein Lösungsmittel im Reaktionsgemisch enthalten ist, so kann dieses durch Destillation im wesentlichen entfernt werden. Bevorzugt wird gegebenenfalls enthaltenes Lösungsmittel nach Wäsche und/oder Neutralisation aus dem Reaktionsgemisch entfernt, falls gewünscht kann dieses aber auch vor der Wäsche beziehungsweise Neutralisation erfolgen.

Dazu kann das Reaktionsgemisch mit einer derartigen Menge an Lagerstabilisator versetzt werden, daß nach Abtrennung des Lösungsmittels 100 - 500, bevorzugt 200 - 500 und besonders bevorzugt 200 - 400 ppm davon im Zielester (Rückstand) enthalten sind.

Die destillative Abtrennung der Hauptmenge an gegebenenfalls verwendetem Lösungsmittel oder niedrigsiedenden Nebenprodukten erfolgt beispielsweise in einem Rührkessel mit Doppelwandheizung und/oder innenliegenden Heizschlangen unter vermindertem Druck, beispielsweise bei 20 - 700 mbar, bevorzugt 30 bis 500 und besonders bevorzugt 50 - 150 mbar und einer Temperatur von 40 - 120 °C.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 - 700 mbar, bevorzugt 30 bis 500 und besonders bevorzugt 50 - 150 mbar und einer Temperatur von 40 - 80 °C durch den Apparat geführt.

Vorteilhaft kann ein unter den Reaktionsbedingungen inertes Gas in den Destillationsapparat eingeleitet werden, beispielsweise 0,1 - 1, bevorzugt 0,2 - 0,8 und besonders bevorzugt 0,3 - 0,7 m³ sauerstoffhaltiges Gas pro m³ Reaktionsgemisch und Stunde.

Der Restlösungsmittelgehalt im Rückstand beträgt nach der Destillation in der Regel unter 5 Gew%, bevorzugt 0,5 - 5 % und besonders bevorzugt 1 bis 3 Gew%.

Das abgetrennte Lösungsmittel wird kondensiert und bevorzugt wiederverwendet.

Falls erforderlich kann zusätzlich oder anstelle der Destillation eine Lösungsmittelstrippung i) durchgeführt werden.

Dazu wird das Produkt, das noch geringe Lösungsmittelmengen oder niedrigsiedende Verunreinigungen enthalten kann, auf 50 - 150 °C, bevorzugt 80 - 150 °C erwärmt und die restlichen Lösungsmittelmengen mit einem geeigneten Gas in einer geeigneten Apparatur entfernt. Zur Unterstützung kann gegebenenfalls auch ein Vakuum angelegt werden.

Geeignete Apparaturen sind beispielsweise Kolonnen von an sich bekannter Bauart, die die üblichen Einbauten, z.B. Böden, Schüttungen oder gerichtete Packungen, bevorzugt Schüttungen aufweisen. Als Kolonneneinbauten kommen prinzipiell alle gängigen Einbauten in Betracht, beispielsweise Böden, Packungen und/oder Füllkörper. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten, bevorzugt.

Denkbar ist hier auch ein Fallfilm-, Dünnfilm- oder Wischfilmverdampfer, wie z.B. ein Luwa-, Rotafilm- oder Sambayverdampfer, der als Spritzschutz beispielsweise mit einem Demister ausgerüstet sein kann.

Geeignete Gase sind unter den Strippbedingungen inerte Gase insbesondere solche, die auf 50 bis 100 °C temperiert sind.

Die Strippgasmenge beträgt beispielsweise 5 - 20, besonders bevorzugt 10 - 20 und ganz besonders bevorzugt 10 bis 15 m³ Strippgas pro m³ Reaktionsgemisch und Stunde.

Falls notwendig kann das Veresterungsgemisch in einem beliebigen Stadium des Aufarbeitungsverfahrens, bevorzugt nach Wäsche/Neutralisation und gegebenenfalls erfolgter Lösungsmittelentfernung einer Filtration j) unterworfen werden, um ausgefallene Spuren an Salzen sowie gegebenenfalls enthaltenem Entfärbungsmittel zu entfernen. Auf eine Vor- oder Nachwäsche e) oder g) wird bevorzugt verzichtet, lediglich ein Filtrationsschritt j) kann sinnvoll sein. Ebenfalls wird auf eine Neutralisation f) bevorzugt verzichtet.

Die Abfolge der Schritte e)/g), sowie h) und j) ist dabei beliebig.

Die nach diesem Verfahren erhältlichen hochfunktionellen, hoch- oder hyperverzweigten Polyester zeichnen sich durch besonders geringe Anteile an Verfärbungen und Verharzungen aus.

Die erfindungsgemäßen Polyester haben ein Molekulargewicht Mₙ von mindestens 500, bevorzugt mindestens 600 und besonders bevorzugt 750 g/mol. Die obere Grenze des Molekulargewichts Mₙ ist bevorzugt 100.000 g/mol, besonders bevorzugt beträgt es nicht mehr als 80.000 und ganz besonders bevorzugt nicht mehr als 30.000 g/mol. Die Angaben zur Polydispersität sowie zum zahlenmittleren und gewichtsmittleren Molekulargewicht Mₙ und M_{w} beziehen sich hier auf gelpermeationschromatographische Messungen, wobei Polymethylmethacrylat als Standard und Tetrahydrofuran, Dimethylacetamid oder Hexafluorisopropanol als Elutionsmittel verwendet wurden. Die Methode ist im Analytiker Taschenbuch Bd. 4, Seiten 433 bis 442 , Berlin 1984 beschrieben.

Die Polydispersität der Polyester beträgt 1,2 bis 50, bevorzugt 1,4 bis 40, besonders bevorzugt 1,5 bis 30 und ganz besonders bevorzugt bis 10.

Die Polyester sind üblicherweise sehr gut löslich, d.h. man kann bei 25 °C klare Lösungen mit einem Gehalt bis zu 50 Gew.-%, in einigen Fällen sogar bis zu 80 Gew.-%, der erfindungsgemäßen Polyester in Tetrahydrofuran (THF), Ethylacetat, n-Butylacetat, Ethanol und zahlreichen anderen Lösemitteln darstellen, ohne dass mit bloßem Auge Gelpartikel detektierbar sind. Dies zeigt den geringen Vernetzungsgrad der erfindungsgemäßen Polyester.

Die hochfunktionellen hoch- und hyperverzweigten Polyester sind carboxyterminiert, carboxy- und Hydroxylgruppen-terminiert und vorzugsweise Hydroxylgruppen-terminiert.

In einer bevorzugten Ausführung sind die hochverzweigten Polyester ganz oder teilweise mit linearen oder verzweigten C₄- bis C₄₀-Alkyl-und/oder -Alkenylresten substituiert. Im Rahmen der vorliegenden Erfindung können Alkenylreste einfach oder mehrfach ungesättigt sein.

Substitution im Rahmen der vorliegenden Erfindung bedeutet, dass die hochverzweigten Polyester während und/oder nach der Polymerisationsreaktion mit Verbindungen A umgesetzt werden. Verbindungen A sind dadurch gekennzeichnet, dass sie einen linearen oder verzweigten C₄- bis C₄₀-Alkyl-und/oder Alkenylrest und eine reaktive Gruppe enthalten. Eine reaktive Gruppe der Verbindung A ist in der Lage, mit dem hochverzweigten Polyester zu reagieren. Bevorzugt enthalten Verbindungen A genau einen linearen oder verzweigten C₄- bis C₄₀-Alkyl-und/oder Alkenylrest und genau eine reaktive Gruppe.

Hochverzweigte Polyester, die mit Verbindungen A umgesetzt wurden, werden als substituierte hochverzweigte Polyester bezeichnet.

Die Substitution kann vollständig oder teilweise erfolgen. Das heißt im Fall der vollständigen Substitution, dass die reaktiven Gruppen des hochverzweigten Polyesters vollständig mit Verbindungen A reagiert haben. Im Fall der teilweisen Substitution haben nicht alle reaktiven Gruppen des hochverzweigten Polyesters mit Verbindungen A reagiert.

Bevorzugt sind die hochverzweigten Polyester mit Octyl- (Capryl-), Nonyl-, Decyl-(Caprinyl-), Undecyl-, Dodecyl- (Laurinyl-), Tetradecyl-, Hexadecyl- (Palmityl-), Heptadecyl-, Octadecyl- (Stearyl-) Resten und/oder den entsprechenden einfach oder mehrfach ungesättigten Äquivalenten, wie zum Beispiel mit Dodecenyl-, Hexadienyl- (Sorbinyl-), Octadecenyl-(Oleyl-), Linolyl- oder Linolenyl-Resten substituiert.

Unter Äquivalent ist in diesem Zusammenhang ein Kohlenwasserstoffrest zu verstehen, der sich von dem entsprechenden linearen oder verzweigten Alkylrest lediglich dadurch unterscheidet, dass er wenigstens eine Doppelbindung aufweist.

Die substituierten hochverzweigten Polyester werden bevorzugt erhalten, indem der erhaltene hochfunktionelle, hoch- oder hyperverzweigte Polyester mit einem geeigneten Funktionalisierungsreagenz, welches mit den OH- und/oder Ester-Gruppen des Polyesters reagieren kann, umgesetzt wird.

Hydroxylgruppen enthaltende hochfunktionelle, hochverzweigte Polyester können zum Beispiel durch Zugabe von Säurederivatgruppen wie Ester, Anhydride oder Amide oder Isocyanatgruppen enthaltenden Molekülen modifiziert werden. Beispielsweise lassen sich Säuregruppen enthaltende Polyester durch Umsetzung mit Anhydridgruppen enthaltenden Verbindungen erhalten.

Dabei beträgt das molare Verhältnis der reaktiven Gruppen der Substitutionsverbindung zu den reaktiven Gruppen des hochverzweigten Polyesters von 1:10 bis 1:1, bevorzugt von 1:5 bis 1:1,1, insbesondere bevorzugt von 1:2 bis 1:1,2. Ein besonders bevorzugter Bereich ist 1:1,7 bis 1:1,4.

In einer bevorzugten Ausführungsform der vorliegenden Erfindung erfolgt die Substitution mit einem Carbonsäurederivat der Formel R-CO-Y und/oder einem Isocyanat der Formel R-NCO, wobei die Reste die nachstehende Bedeutung haben.

R ist lineares oder verzweigtes C₄- bis C₄₀-Alkyl.

Y ist OR¹, OC(O)R² oder NR³₂. Dabei ist R¹ Wasserstoff oder lineares oder verzweigtes C₁- bis C₆-Alkyl, R² lineares oder verzweigtes C₄- bis C₄₀-Alkyl, wobei R und R² gleich oder verschieden sein können. R³ ist Wasserstoff oder lineares oder verzweigtes C₁-bis C₄-Alkyl, wobei die beiden Reste R³ gleich oder verschieden voneinander sein können.

Bevorzugte Verbindungen sind lineare C₄-C₄₀-Alkylisocyanate, besonders bevorzugt sind Octyl- (Capryl-) isocyanat, Nonylisocyanat, Decyl- (Caprinyl-) isocyanat, Undecylisocyanat, Dodecyl- (Laurinyl-) isocyanat, Tetradecylisocyanat, Hexadecyl- (Palmityl-) isocyanat, Heptadecylisocyanat, Octadecyl- (Stearyl-) isocyanat.

Weitere bevorzugte Verbindungen sind lineare C₄-C₄₀-Alkenylisocyanate mit einer oder mehreren Doppelbindungen, besonders bevorzugt sind Dodecenyl-, Hexadienyl- (Sorbinyl-), Octadecenyl-(Oleyl-), Linolyl- oder Linolenyl-isocyanat.

Eine ganz besonders bevorzugte Verbindung ist Stearylisocyanat.

Die Substitution kann beispielsweise in einem nachträglichen Verfahrensschritt (Schritt c)) erfolgen. Die Substitution kann aber auch bereits während der Herstellung der hochverzweigten Polyester erfolgen.

Bevorzugt erfolgt die Substitution in einem nachträglichen Verfahrensschritt.

Erfolgt die Substitution in einem nachfolgenden Verfahrensschritt, so wird bevorzugt der hochverzweigte Polyester vorgelegt und eine oder mehrere Verbindungen A werden zugegeben.

Die Substitution erfolgt üblicherweise bei einer Temperatur von 0 bis 300 °C, bevorzugt 0 bis 250°C, besonders bevorzugt bei 60 bis 200°C und ganz besonders bevorzugt bei 60 bis 160°C in Substanz oder in Lösung. Dabei können allgemein alle Lösungsmittel verwendet werden, die gegenüber den jeweiligen Edukten inert sind. Bevorzugt verwendet werden organische Lösungsmittel, wie zum Beispiel Decan, Dodecan, Benzol, Toluol, Chlorbenzol, Xylol, Dimethylformamid, Dimethylacetamid oder Solventnaphtha.

In einer bevorzugten Ausführungsform wird die Substitutionsreaktion in Substanz durchgeführt. Bei der Reaktion freiwerdende niedermolekulare Verbindungen können zur Beschleunigung der Reaktion aus dem Reaktionsgleichgewicht entfernt werden, zum Beispiel destillativ, gegebenenfalls bei vermindertem Druck.

Zur Vervollständigung der Reaktion kann es nötig sein, die Temperatur des Reaktionsbehälters nach der Zugabe der Verbindung A beziehungsweise, falls mehrere voneinander verschiedene Verbindungen A eingesetzt werden, nach der Zugabe der Verbindungen A anzuheben. Üblicherweise beträgt die Anhebung 10 bis 50°C, bevorzugt beträgt sie 20 bis 40°C.

Die Substitution der hochfunktionellen Polyester erfolgt zumeist in einem Druckbereich von 0,1 mbar bis 20 bar, bevorzugt bei 1 mbar bis 5 bar, in Reaktoren oder Reaktorkaskaden, die im Batchbetrieb, halbkontinuierlich oder kontinuierlich betrieben werden.

Erfindungsgemäß ist die Verwendung eines substituierten hochverzweigten Polyesters als Verdicker in kosmetischen und/oder dermatologischen Formulierungen.

Bevorzugt ist die Verwendung in hautkosmetischen Formulierungen.

Bevorzugt wird die Verwendung als Ölverdicker .

Die kosmetische Zusammensetzung enthält bevorzugt mindestens einen kosmetisch geeigneten Träger.

### Hautkosmetische Zubereitungen

Die hautkosmetische Zusammensetzungen, insbesondere solche zur Pflege der Haut, können in verschiedenen Formen vorliegen und eingesetzt werden. So können sie z. B. eine Emulsion vom Typ Öl-in-Wasser (O/W) oder eine multiple Emulsion, beispielsweise vom Typ Wasser-in-ÖI-in-Wasser (W/O/W). Auch emulgatorfreie Formulierungen wie Hydrodispersionen, Hydrogele oder eine Pickering-Emulsion sind vorteilhafte Ausführungsformen.

Die Konsistenz der Formulierungen kann von pastösen Formulierungen über fließfähige Formulierungen bis hin zu dünnflüssigen, sprühbaren Produkten reichen. Dementsprechend können Cremes, Lotionen oder Sprays formuliert werden. Zur Anwendung werden die erfindungsgemäßen kosmetischen Zusammensetzungen in der für Kosmetika und Dermatika üblichen Weise auf die Haut in ausreichender Menge aufgebracht.

Der Salzgehalt der Hautoberfläche ist ausreichend, um die Viskosität der erfindungsgemäßen Zubereitungen derart zu erniedrigen, dass eine einfache Verteilung und Einarbeitung der Zubereitungen ermöglicht wird.

Die hautkosmetischen Zubereitungen liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weitere vorteilhafte hautkosmetische Zubereitungen sind Gesichtswasser, Gesichtsmasken, Deodorantien und andere kosmetische Lotionen und Zubereitungen für die dekorative Kosmetik, beispielsweise Abdeckstifte, Theaterfarbe, Mascara, Lidschatten, Lippenstifte, Kajalstifte, Eyeliner, Makeups, Grundierungen, Rouges, Puder und Augenbrauenstifte.

Außerdem können die Zusammensetzungen verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Die erfindungsgemäßen hautkosmetischen Zubereitungen enthalten neben dem W/W-Emulsionspolymerisat und geeigneten Trägern noch weitere in der Kosmetik übliche Wirk- und/oder Hilfsstoffe, wie zuvor und nachfolgend beschrieben.

Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, natürliche und synthetische Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Konditioniermittel, Rückfetter und weitere übliche Additive.

Den Zusammensetzungen können auch weitere Polymere zugesetzt werden, falls spezielle Eigenschaften eingestellt werden sollen. Zur Einstellung bestimmter Eigenschaften wie z. B. Verbesserung des Anfassgefühls, des Spreitverhaltens, der Wasserresistenz und/oder der Bindung von Wirk- und Hilfs-Stoffen wie Pigmenten, können die Zusammensetzungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Weitere mögliche Inhaltsstoffe der Zusammensetzungen sind nachfolgend unter dem jeweiligen Stichwort beschrieben.

### Öle, Fette und Wachse

Die haut- und haarkosmetischen Zusammensetzungen enthalten bevorzugt auch Öle, Fette oder Wachse.

Bestandteile der Öl- und/oder Fettphase der kosmetischen Zusammensetzungen werden vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat Dicaprylyl Carbonat (Cetiol CC) und Cocoglyceride (Myritol 331), Butylen Glycol Dicaprylat/Dicaprat und Dibutyl Adipat sowie synthetische, halbsynthetische und natürliche Gemische solcher Ester, wie z.B. Jojobaöl.

Ferner können eine oder mehrere Olkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Erfindungsgemäß vorteilhaft wird die Olkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Erfindungsgemäß vorteilhaft sind Mischungen aus C12-C15-Alkylbenzoat und 2-Ethylhexylisostearat, Mischungen aus C12-C15-Alkylbenzoat und Isotridecylisononanoat sowie Mischungen aus C12-C15-Alkylbenzoat, 2-Ethylhexylisostearat und Isotridecylisononanoat.

Erfindungsgemäß besonders bevorzugt werden als Öle mit einer Polarität von 5 bis 50 mN/m Fettsäuretriglyceride, insbesondere Sojaöl und/oder Mandelöl eingesetzt.

Von den Kohlenwasserstoffen sind vorteilhaft Paraffinöl, Squalan, Squalen und insbesondere Polyisobutene, die auch hydriert sein können, im Sinne der vorliegenden Erfindung zu verwenden.

Ferner kann die Ölphase vorteilhaft gewählt werden aus der Gruppe der Guerbetalkohole. Guerbetalkohole entstehen nach der Reaktionsgleichung 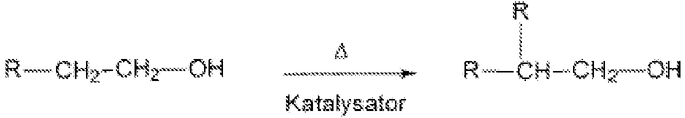 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in kosmetischen Zusammensetzungen eingesetzt werden.

Die Verwendung von Guerbet-Alkoholen in Kosmetika ist an sich bekannt. Solche Species zeichnen sich dann meistens durch die Struktur 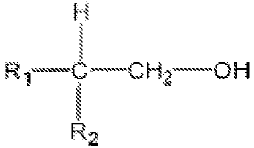 aus. Dabei bedeuten R₁ und R₂ in der Regel unverzweigte Alkylreste. Erfindungsgemäß vorteilhaft werden der oder die Guerbet-Alkohole gewählt aus der Gruppe, wobei
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind 2-Butyloctanol (beispielsweise als Isofol^{®}12 (Condea) kommerziell erhältlich) und 2-Hexyldecanol (beispielsweise als Isofol^{®}16 (Condea) kommerziell erhältlich).

Auch Mischungen von erfindungsgemäßen Guerbet-Alkoholen sind erfindungsgemäß vorteilhaft zu verwenden wie beispielsweise Mischungen aus 2-Butyloctanol und 2-Hexyldecanol (beispielsweise als Isofol^{®}14 (Condea) kommerziell erhältlich).

Auch beliebige Abmischungen solcher Öl- und Wachskomponenten sind vorteilhaft im Sinne der vorliegenden Erfindung einzusetzen. Unter den Polyolefinen sind Polydecene die bevorzugten Substanzen.

Vorteilhaft kann die Olkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Niedermolekulare Silicone oder Siliconöle sind in der Regel durch folgende allgemeine Formel definiert

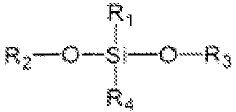

Höhermolekulare Silicone oder Siliconöle sind in der Regel durch folgende allgemeine Formel definiert 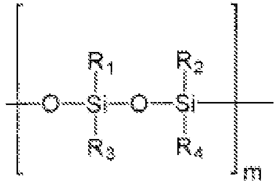 wobei die Siliciumatome mit gleichen oder unterschiedlichen Alkylresten und/oder Arylresten substituiert sein können, welche hier verallgemeinernd durch die Reste R₁ bis R₄ dargestellt sind. Die Anzahl der unterschiedlichen Reste ist aber nicht notwendigerweise auf bis zu 4 beschränkt. m kann dabei Werte von 2 bis 200.000 annehmen.

Erfindungsgemäß vorteilhaft einzusetzende cyclische Silicone sind in der Regel durch folgende allgemeine Formel definiert 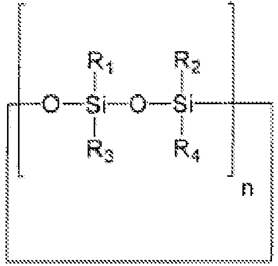 wobei die Siliciumatome mit gleichen oder unterschiedlichen Alkylresten und/oder Arylresten substituiert werden können, welche hier verallgemeinernd durch die Reste R₁ bis R₄ dargestellt sind. Die Anzahl der unterschiedlichen Reste ist aber nicht notwendigerweise auf bis zu 4 beschränkt. n kann dabei Werte von 3/2 bis 20 annehmen. Gebrochene Werte für n berücksichtigen, daß ungeradzahlige Anzahlen von Siloxylgruppen im Zyklus vorhanden sein können.

Vorteilhaft wird Phenyltrimethicon als Siliconöl gewählt. Auch andere Silikonöle, beispielsweise Dimethicon, Hexamethylcyclotrisiloxan, Phenyldimethicon, Cyclomethicon (z.B. Decamethylcyclopentasiloxan), Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden. Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxanpolyalkyl-Polyether-copolymere wie z.B. Cetyl-Dimethicon-Copolyol. Vorteilhaft wird Cyclomethicon (Octamethylcyclotetrasiloxan) als erfindungsgemäß zu verwendendes Silikonöl eingesetzt.

Vorteilhaft zu verwendende Fett- und/oder Wachskomponenten können aus der Gruppe der pflanzlichen Wachse, tierischen Wachse, Mineralwachse und petrochemischen Wachse gewählt werden. Vorteilhaft sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise Syncrowax^{®}HRC (Glyceryltribehenat), und Syncrowax^{®}AW 1 C (C₁₈₋₃₆-Fettsäure) sowie Montanesterwachse, Sasolwachse, hydrierte Jojobawachse, synthetische oder modifizierte Bienenwachse (z. B. Dimethicon Copolyol Bienenwachs und/oder C₃₀₋₅₀-Alkyl Bienenwachs), Cetyl Ricinoleate wie beispielsweise Tegosoft^{®}CR, Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride wie beispielsweise Hydriertes Soy Glycerid, Trihydroxystearin, Fettsäuren, Fettsäureester und Gly kolester wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan. Erfindungsgemäß können die Fett- und/oder Wachskomponenten sowohl einzeln als auch als Gemisch in den Zusammensetzungen verwendet werden.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprin-säure-triglycerid, Dicaprylylether. Besonders vorteilhaft sind Mischungen aus Octyldodecanol, Capryl-Caprinsäure-triglycerid, Dicaprylylether, Dicaprylyl Carbonat, Cocoglyceriden oder Mischungen aus C₁₂₋₁₅-Alkylbenzoat und 2-Ethylhexylisostearat, Mischungen aus C₁₂₋₁₅-Alkylbenzoat und Butylen Glycol Dicaprylat/Dicaprat sowie Mischungen aus C₁₂₋₁₅-Alkylbenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Die Ölkomponente kann auch vorteilhaft aus der Gruppe der Phospholipide gewählt werden. Die Phospholipide sind Phosphorsäureester acylierter Glycerine. Von größter Bedeutung unter den Phosphatidylcholinen sind beispielsweise die Lecithine, welche sich durch die allgemeine Struktur 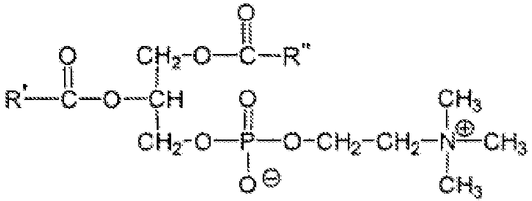 auszeichnen, wobei R' und R" typischerweise unverzweigte aliphatische Reste mit 15 oder 17 Kohlenstoffatomen und bis zu 4 cis-Doppelbindungen darstellen.
Als erfindungsgemäß vorteilhaftes Paraffinöl kann erfindungsgemäß Merkur Weissoel Pharma 40 von Merkur Vaseline, Shell Ondina^{®} 917, Shell Ondina^{®}927, Shell
Oil 4222, Shell Ondina^{®}933 von Shell & DEA Oil, Pionier^{®} 6301 S, Pionier^{®} 2071 (Hansen & Rosenthal) eingesetzt werden.
Geeignete kosmetisch verträgliche Öl- und Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier in vollem Umfang Bezug genommen wird.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstands nicht nur in der jeweils angegebenen Kombination, sondern auch in anderen Kombinationen verwendbar sind, ohne den Rahmen der Erfindung zu verlassen.

Die vorliegende Erfindung soll anhand der nachfolgenden Beispiele näher erläutert werden.

### Beispiele

### Messmethoden

### Die IR-Messungen erfolgten mit einem Nicolet 210-Gerät.

Die Bestimmung der Säurezahl und der Hydroxylzahl erfolgte nach DIN 53240, Teil 2. Die Bestimmung desMolekulargewichts erfolgte mit Hilfe von Gelpermeationschromatographie mit einem Refraktometer als Detektor. Als mobile Phase wurde Dimethylacetamid verwendet, als Standard zur Bestimmung des Molekulargewichts wurde Polymethylmethacrylat (PMMA) eingesetzt.

### Einsatzstoffe

DBTL: Dibutylzinn-dilaurat, Hersteller: Fa. Sigma-Aldrich
Paraffinöl: Nujol, Fluka AG

### Beispiel 1: Herstellung eines hochverzweigten Polyesters

233,2 g Adipinsäure (1.6 mol) und 266,0 g (1,33 mol) eines Triols auf Basis Trimethylolpropan, das statistisch mit 1,2 Propylenoxideinheiten verethert wurde, wurden in einem 1000-mL-Glasreaktor, ausgestattet mit Rührer, Rückflusskühler, Gaseinlass, daran angeschlossen ein Vakuumsystem mit zwischengeschalteter Kühlfalle und Innenthermometer vorgelegt. Nach Zugabe von 200 ppm DBTL wurde die Mischung auf 150°C erwärmt. Dabei wurde der Innendruck auf einen Endwert von ca. 10 mbar so abgesenkt, dass das entstehende Wasser kontrolliert entfernt wurde. 5,5 Stunden wurde bei dieser Temperatur gerührt. Die Säurezahl betrug 68 mg KOH/g. 97 g des o.g. Triols (0,8 Äquivalente pro Säuregruppe) wurden zur Reaktionsmischung hinzugegeben. Die Reaktionsmischung wurde weitere fünf Stunden bei einem Innendruck von ca. 10 mbar gerührt und anschließend bei diesem Druck auf Umgebungstemperatur abgekühlt.
Das Endprodukt wurde als viskose, klare Flüssigkeit erhalten, die folgende Eigenschaften aufweist: Säurezahl = 26 mg KOH/g; Hydroxylzahl = 204 mg KOH/g; Viskosität: 6800 mPas (75°C)

### Beispiele 2 - 8: Modifizierung des hochverzweigten Polyesters mit Stearylisocyanat

Hochverzweigter Polyester aus Beispiel 1 wurde in einem 250-mL-Glasreaktor, ausgestattet mit Rührer, Rückflusskühler, Gaseinlass, Innenthermometer und Tropftrichter, der die benötigte Menge Stearylisocyanat enthielt, vorgelegt. Die verwendeten Mengen an hochverzweigtem Polyester und Stearylisocyanat sind der nachfolgenden Tabelle zu entnehmen.

Der Reaktor wurde auf 80°C erwärmt und das Isocyanat wurde innerhalb von 15 Minuten zugetropft. Danach wurde die Reaktionsmischung weitere zwei Stunden bei 120°C gerührt und der Reaktionsfortschritt durch das Verschwinden der Isocyanatgruppen mit Hilfe von IR-Spektroskopie verfolgt (Verschwinden der Isocyanat-Bande bei 2270 cm⁻¹).

| Beispiel | 2 | 3 | 4 | 5 | 6 | 7 | 8 |
|---|---|---|---|---|---|---|---|
| Hochverzweigter Polyester (Beispiel 1) [g] | 50 | 50 | 51,2 | 76,6 | 50 | 50 | 50 |
| Mol% NCO | 40 | 50 | 60 | 70 | 80 | 90 | 100 |
| Menge Stearylisocyanat [g] | 21,5 | 26,9 | 33,1 | 58 | 43,04 | 48,4 | 53,8 |

### Beispiel 9: Gelbildung durch Zugabe der Stearyl-modifizierten hochverzweigten Polyester zu Paraffinöl

Verschiedene Mengen (0,5 bis 20 Gewichts-%) der Polymere der Beispiele 3 bis 8 wurden in Paraffinöl gelöst. Die Konzentration, bei der eine sichtbare Gelbildung eintrat, wird in der folgenden Tabelle angegeben:

| Polymer aus Beispiel ... | 3 | 4 | 5 | 6 | 7 | 8 |
|---|---|---|---|---|---|---|
| Verhältnis NCO/OH [%] | 50 | 60 | 70 | 80 | 90 | 100 |
| Gelbildungskonzentration (%) | 10 | 10 | 7 | 6 | 4 | 4 |

## Patentansprüche

1. Verwendung eines hochverzweigten Polyesters, der ganz oder teilweise mit linearen oder verzweigten C₄- bis C₄₀-Alkyl- oder Alkenylresten substituiert ist, als Verdicker in kosmetischen und/oder dermatologischen Formulierungen.

2. Verwendung nach Anspruch 1, **dadurch gekennzeichnet, dass** die Substitution mit einem Derivat der Formel R-CO-Y und/oder R-NCO erfolgt
mit
R = lineares oder verzweigtes C₄- bis C₄₀-Alkyl,
Y = OR¹, OC(O)R², NR³₂ oder Halogen
R¹ = Wasserstoff, lineares oder verzweigtes C₁- bis C₆-Alkyl,
R² = lineares oder verzweigtes C₄- bis C₄₀-Alkyl, wobei R und R² gleich oder verschieden sein können,
R³ = Wasserstoff, lineares oder verzweigtes C₁- bis C₄-Alkyl, wobei die beiden Reste R³ gleich oder verschieden voneinander sein können.

3. Verwendung nach Anspruch 1 oder 2, **dadurch gekennzeichnet, dass** die kosmetische Formulierung mindestens einen kosmetisch geeigneten Träger enthält.

4. Verwendung nach Anspruch 1 in hautkosmetischen Formulierungen.

5. Verwendung nach einem der Ansprüche 1 bis 4 als Ölverdicker.

## Claims

1. The use of a highly branched polyester which is completely or partly substituted by linear or branched C₄- to C₄₀-alkyl- or alkenyl radicals, as thickener in cosmetic and/or dermatological formulations.

2. The use according to claim 1, wherein the substitution takes place with a derivative of the formula R-CO-Y and/or R-NCO
where
R = linear or branched C₄- to C₄₀-alkyl,
Y = OR¹, OC(O)R², NR³₂ or halogen
R¹ = hydrogen, linear or branched C₁- to C₆-alkyl,
R² = linear or branched C₄- to C₄₀-alkyl, where R and R² may be identical or different,
R³ = hydrogen, linear or branched C₁- to C₄-alkyl, where the two radicals R³ may be identical or different from one another.

3. The use, according to claim 1 or 2, wherein the cosmetic formulation comprises
at least one cosmetically suitable carrier.

4. The use according to claim 1 in skin cosmetic formulations.

5. The use according to any one of claims 1 to 4 as oil thickener.

## Revendications

1. Utilisation d'un polyester hautement ramifié, qui est totalement ou partiellement substitué par des radicaux alkyle ou alcényle en C₄-C₄₀ linéaires ou ramifiés, en tant qu'épaississant dans des formulations cosmétiques et/ou dermatologiques.

2. Utilisation selon la revendication 1, **caractérisée en ce que** la substitution s'effectue avec un dérivé de formule R-CO-Y et/ou R-NCO
où
R = un groupe alkyle en C₄-C₄₀ linéaire ou ramifié,
Y = OR¹, OC(O)R², NR³₂ ou un atome d'halogène,
R¹ = un atome d'hydrogène, un groupe alkyle en C₁-C₆ linéaire ou ramifié,
R² = un groupe alkyle en C₄-C₄₀ linéaire ou ramifié, R et R² pouvant être identiques ou différents,
R³ = un atome d'hydrogène, un groupe alkyle en C₁-C₄ linéaire ou ramifié, les deux radicaux R³ pouvant être identiques ou différents.

3. Utilisation selon la revendication 1 ou 2, **caractérisée en ce que** la formulation cosmétique contient au moins un véhicule cosmétiquement approprié.

4. Utilisation selon la revendication 1, dans des formulations cosmétiques pour la peau.

5. Utilisation selon l'une quelconque des revendications 1 à 4, en tant qu'épaississant d'huile.
